# EUROPEAN PATENT SPECIFICATION

(11) **EP 2 084 181 B1**
(45) Date of publication and mention of the grant of the patent: **23.11.2011**
(21) Application number: 07834893.5
(22) Date of filing: 12.09.2007
(51) Int. Cl.: C07K 14/475, C07K 14/47, G01N 33/68, A61K 38/18, C07K 16/26

(54) **A METHOD AND PREPARATIONS FOR THE DIAGNOSIS AND THERAPY OF MULTIPLE SCLEROSIS AND IMMUNE DEMYELINATING POLYNEUROPATHY**
VERFAHREN UND ZUBEREITUNGEN ZUR DIAGNOSE UND THERAPIE VON MULTIPLER SKLEROSE UND INFLAMMATORISCHER DEMYELINISIERENDER POLYNEURORADIKULITIS
PROCÉDÉ ET PRÉPARATIONS POUR LE DIAGNOSTIC ET LE TRAITEMENT DE LA SCLÉROSE EN PLAQUES ET DE LA POLYNEUROPATHIE DÉMYÉLINISANTE D'ORIGINE IMMUNOLOGIQUE

(30) Priority: 12.09.2006 PL 38062006
(43) Date of publication of application: 05.08.2009
(73) Proprietor: JUREWICZ, Anna, 94-118 Lodz (PL); MATYSIAK, Mariola, 95-050 Konstantynow Lodzki (PL); SELMAJ, Krzysztof, 90-158 Lodz (PL)
(72) Inventor: JUREWICZ, Anna, 94-118 Lodz (PL); MATYSIAK, Mariola, 95-050 Konstantynow Lodzki (PL); SELMAJ, Krzysztof, 90-158 Lodz (PL)
(74) Representative: Witek, Rafal
(86) International application number: PCT/PL2007/000065
(87) International publication number: WO 2008/033044

(56) References cited:
- WO-A-2006/047049
- REINDL M ET AL.: "Serum and cerebrospinal fluid antibodies to Nogo-A in patients with multiple sclerosis and acute neurological disorders" JOURNAL OF NEUROIMMUNOLOGY, vol. 145, 2003, pages 139-147, XP002465416
- HU F ET AL.: "Nogo-A Interacts with the Nogo-66 Receptor through Multiple Sites to Create an Isoform-Selective Subnanomolar Agonist" THE JOURNAL OF NEUROSCIENCE, vol. 25, no. 22, 1 June 2005 (2005-06-01), pages 5298-5304, XP002465417
- DATABASE EMBL [Online] 8 November 2005 (2005-11-08), OHIRA M ET AL.: "A large-scale cloning from oligo-capping cDNA libraries of primary neuroblastoma" XP002465419 accession no. Q3LIF4
- JUREWICZ A ET AL.: "Soluble Nogo-A, an inhibitor of axonal regeneration, as a biomarker for multiple sclerosis" NEUROLOGY, vol. 68, 23 January 2007 (2007-01-23), pages 283-287, XP002465418

### SEQUENCE LISTING

<110> JUREWICZ, Anna
   MATYSIAK , Mariola
   SELMAJ , Krzysztof
<120> A method and preparations for the diagnosis and therapy of multiple sclerosis and immune demyelinating polyneuropathy
<130> PZ/0158/RW/PCT
<150> P.380606
   <151> 2006-09-12
<160> 1
<170> PatentIn version 3.3
<210> 1
   <211> 18
   <212> PRT
   <213> homo sapiens
<400> 1 
Phe Ser Lys Leu Ala Arg Glu Tyr Thr Asp Leu Glu Val Ser His Lys
1               5                   10                  15
 Ser Glu

## Description

### Field of the invention

The subject of the present invention is a method of diagnosing or treatment multiple sclerosis and immune demyelinating polyneuropathy as well as preparations used in these methods. The present invention is applicable in medicine.

### State of the art

Multiple sclerosis (SM) is the most frequent demyelinating disorder of the central nervous system. The neurological deficiencies gradually accumulate and the disease irreversibly leads to permanent disability. Until recently, SM was perceived as a disease which causes damage to the myelin sheath. Recently, however, attention has shifted to axonal damage (neuronal cytoplasmic protrusions responsible for signal transduction) with the retention of properly functional myelin (normal-appearing white matter - NAWM). Axonal pathology is perceived as an importan element of the disease, responsible for, amongst others, the occurrence of irreversible and permanent neurological changes, which are the most significant characteristics of the disease.

MS is diagnosed using clinical criteria (McDonald et al. Annals of Neurology, 2002) supplemented by the results of many immunological tests (including of cerebrospinal fluid) and magnetic resonance images. These results, however, are not specific and are only of supplementary value in the diagnosis of MS. There thus exists a need for a more precise MS diagnosis technique.

Proteins of the Nogo group belong to the reticulon protein family. Their initial descriptions are related to descriptions of proteins which inhibit axonal regeneration in the central nervous system (CNS) following mechanical injury. The inability to regenerate is characteristic of the CNS in contrast to the peripheral nervous system (PNS), where damaged axons are "reconstructed". One of the proteins responsible for the inhibition of regeneration in the CNS is Nogo-A. To date three forms of Nogo have been described: A,B and C. It has been shown that Nogo-A occurs primarily in oligodendrocytes, myelin-producing glial cells and withing the myelin sheath which incurs damage during MS. Nogo-B is ubiquitous in the organism while Nogo-C is primarily located in muscle. All forms of Nogo (A,B,C) possess a common domain, Nogo-66, which inhibits axonal regeneration through a recently described receptor. Nogo-A, occurring in oligodendrocytes possesses a characteristic fragment, amino-Nogo, which does not occur in the remaining forms of Nogo, and which also inhibits axonal regeneration.

Patent US2003000437931 describes a method of diagnosing a biological sample, i.e. blood, containing the Nogo protein for a tendency for psychological diseases, for example schizophrenia. The patent described makes use of Nogo-A polymorphism in its specific region.

The Nogo-A protein is also of use in the treatment of autoimmune diseases. Patent WO2004 110353 describes a method based on the administration of Nogo-A epitopes in order to stimulate an immune response.

Reindl et al, Journal of Neuroimmunology 145 (2003), pages 139-147, describe antibodies raised against the fragment Nogo-66 from human Nogo-A for use in the detection of multiple sclerosis.

Despite the above described applications, there still exists a need for a test for diagnosing the probability of acquiring MS as well as for its treatment.

### The nature of the present invention

The subject of the present invention is an isolated fragment of the Nogo-A protein with a molecular mass of 20 kDa, containing the peptide sequence FSKLAREYTDLEVSHKSE (SEQ ID No.1).

The next subject of the present invention is also an isolated polynucleotide molecule coding for the fragment of the Nogo-A protein with a molecular mass of 20 kDa, containing the amino-acid sequence of peptide sequence SEQ ID No.1.

The next subject of the present invention is a polynucleotide molecule encoding the polypeptide FSKLAREYTDLEVSHKSE (SEQ ID No.1).

The next subject of the present invention is a method of diagnosing multiple sclerosis or immune demyelinating polyneuropathy, characterized in that a sample of a patient's cerebrospinal fluid is tested for the presence of the Nogo-A protein fragment with a molecular mass of 20 kDa and possessing the amino-acid sequence containing the sequence of peptide SEQ ID No.1, wherein its presence is evidence of multiple sclerosis or immune demyelinating polyneuropathy. Preferentially, the method according to the present invention is characterized in that the diagnosis of the presence of the Nogo-A protein fragment makes use of an antibody specific for said fragment.

The next subject of the present invention is a method of obtaining antibodies for the detection or treatment of multiple sclerosis or immune demyelinating polyneuropathy encompassing the immunisation of a mammal with a specific antigen and the isolation the specific antibodies against the antigen, characterized in that the immunisation is performed using the Nogo-A protein fragment with a molecular mass of 20 kDa and possessing the amino-acid sequence containing the sequence of peptide SEQ ID No.1 or a fragment of said protein containing SEQ ID NO.1 preferentially the peptide consisting of SEQ ID No.1.

The next subject of the present invention is an antibody produced using the method according to the present invention for application in the diagnostics or therapy of multiple sclerosis or immune demyelinating polyneuropathy.

The next subject of the present invention is an application of the antibody specific for the Nogo-A protein fragment with a molecular mass of 20 kDa and possessing the amino-acid sequence containing the sequence of peptide SEQ ID No.1 or an antibody specific for a fragment of this protein in the manufacture of a preparation for the treatment or diagnosis of multiple sclerosis or immune demyelinating polyneuropathy. Preferentially, the antibody used is specific against the peptide containing SEQ ID No.1.

The next subject of the present invention is the application of the Nogo-A protein fragment with a molecular mass of 20 kDa and possessing the amino-acid sequence containing the sequence of peptide SEQ ID No.1 or a fragment of said protein containing SEQ ID No.1 in the manufacture of a preparation for the treatment or diagnosis of multiple sclerosis or immune demyelinating polyneuropathy.

Preferentially, the protein fragment used is a peptide consisting of SEQ ID No.1.

To summarise, it should be stated that the present invention relates to the use of the identification of a fragment of the Nogo protein in cerebrospinal fluid in the diagnostics or treatment of multiple sclerosis and immune demyelinating polyneuropathy.

The present invention is based on the unexpected detection of a fragment of the Nogo-A protein with a molecular mass of 20kDa exclusively in the cerebrospinal fluid of patients afflicted with multiple sclerosis and immune demyelinating polyneuropathy. In many other syndromes, neither the occurrence of this protein nor of its fragments were noted in cerebrospinal fluid. The Nogo protein fragment occurs in all clinical stages of multiple sclerosis as well as in patients at different phases of disease activity, and thus may be a viable biomarker of said disease. Its occurrence is not dependent on the duration of the disease, nor on the extent of disability. It is the first diagnostic marker of multiple sclerosis and immune demyelinating polyneuropathy. In known methods, disease recognition is based on clinical signs and MRI results for multiple sclerosis and electroneurography and electromyography for demyelinating polyneuropathy. Whereas in the method according to the present invention, one tests for the Nogo protein fragment in cerebrospinal fluid, which is the basis for the described diagnostic test for both diseases.

### Short descriptions of figures

In order to supplement the application, the description of the subject of the present invention has been supplemented with the following figures:

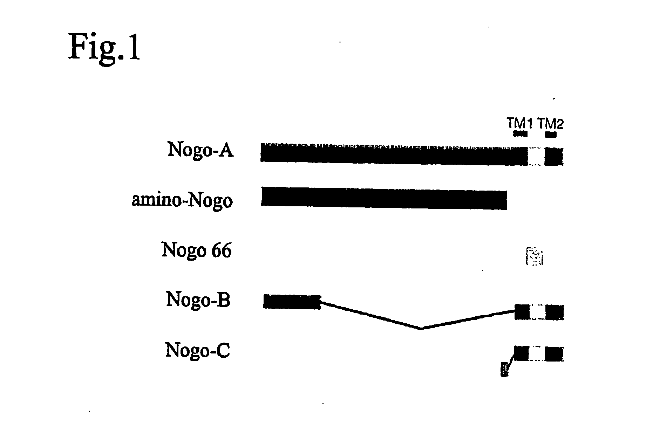

Fig.1. Predicted structures of the Nogo proteins. The full Nogo-A sequence encompasses an N-terminal domain called amin-Nogo as well as the fragment Nogo-66. (Chen et al. Nature, 2000; 403:434-439).

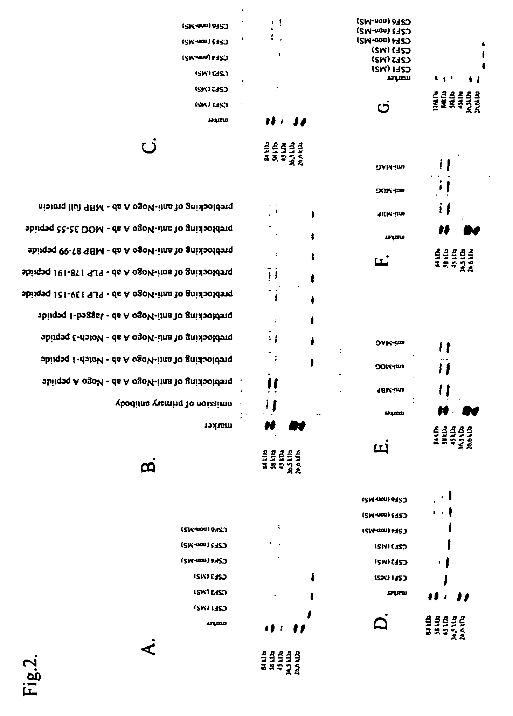

Fig.2. A) A Western blot of samples of cerebrospinal fluid (CSF) in patients afflicted with MS (lanes 1-3) and control patients (lanes 4-6). The detection of the Nogo A fragment with a molecular mass of about 20kDa was performed using a commercially available antibody (Santa Cruz, sc 11032) specific against the N-terminal end of Nogo-A in samples from patients afflicted with MS and a lack of this protein in control patients.
   B) The Nogo A fragment was not detected in CSF samples from MS not treated with the primary antibody (lane 1) nor those containing a peptide which blocks Nogo A (lane 2); signal inhibition was not noted following prior blocking of anti-Nogo-A antibodies with other blocking peptides, namely: Notch-1 (lane 3), Notch-3 (lane 4), Jagged-1(lane 5), PLP 139-151 (lane 6), PLP 178-191 (lane 7), MBP 87-99 (lane 8), MOG 35-55 (lane 9) and full-length MBP (lane 10). C) Absence of immunoreactivity in CSF samples from MS and control patients with an antibody specific against the C-terminal end of the Nogo protein. D) Nogo-R in samples of CSF from patients afflicted with MS and control patients. The two upper bands are evidence of the non-specific binding of the secondary antibody observed in all CSF samples, including the control samples, which is not eliminated through contact with the primary antibody nor by a blocking peptide. E) Absence of MAG, MOG, and MBP detection in CSF samples from MS patients. F) Absence of MAG, MOG and MBP detection in CSF samples from control patients. G.) A Western blot of CSf samples from MS patients (lanes 1-3) and control patients (lanes 4-6). The detection of the Nogo A fragment with a molecular mass of about 20kDa was performed using an antibody (Chemicon AB5888) specific against Nogo-A in samples from patients afflicted with MS and an absence of detection.

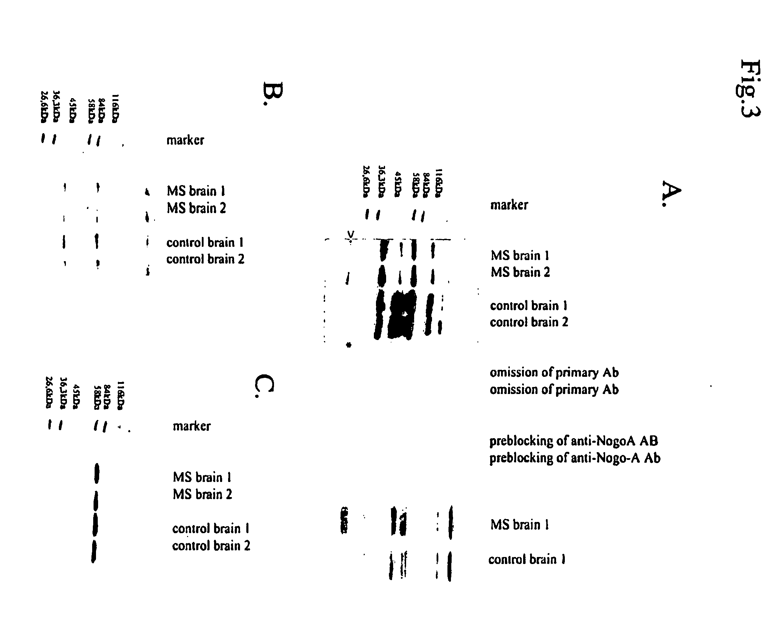

Fig.3. A Western blot of cerebral tissue homogenates from patients afflicted with MS and others. A) The 20 kDa fragment of Nogo A is present in the tissues of MS patients (lanes 1 and 2), but not in control patients(lane 3 and 4), wherein other Nogo-A fragments with a higher molecular mass are present in both MS and control patients. In samples lacking the primary antibody, no Nogo A fragments were observed inclusive of the 20 kDa fragment (lanes 5,6). Similar results were observed in samples supplemented with a peptide blocking the antibody (lanes 7,8). The Nogo A fragment with a molecular mass of about 20kDa was also discovered in the brains of MS patients using various rabbit antibodies specific against Nogo A produced by Chemicon (lane 9) and was not observed in control brain samples (lane 10). B) All three isoforms, Nogo A, B and C were identified using an antibody specific against the C-terminal end of Nogo in brain tissue samples from MS and control patients. C) Nogo-R is present in tissue samples from both MS and control patients.

### Detailed description of the present invention

Since axonal regeneration does not occur in the prains of patients afflicted with MS, the presence of Nogo proteins was evaluated in the CSF of MS patients. A Western blot was used using two antibodies from the Santa-Cruz corporation recognizing: 1.) a fragment characteristic of Nogo-A (Santa Cruz, sc-11032) and 2.) a fragment common to all forms of Nogo (Santa Cruz sc-11033). The presence of a 20 kDa fragment was noted in the CSF of MS patients (94% of MS patient CSF samples) recognized by the anti-Nogo A antibody (Santa Cruz, sc-11032), which was not found in the CSF of patients afflicted with CSF disorders other than multiple sclerosis, including immune diseases (menegitis and cerebral inflammation), degenerative diseases (Creutzfelda-Jakob disease), and cerebral ischaemia (strokes). An identical NogoA fragment occurred in brain lysates of MS patients (autopsy material), but not in lysates of control brains. These results make it possible to state that this fragment is formed directly in the brains of SM patients. Such restrictively specific localisation of the Nogo-A fragment in the CSF of MS patients facilitates the use of this method in SM diagnosis. Over 80 CSF samples from MS patients and over 100 samples from control patients were studied. The extremely high specificity of Nogo-A occurrence in the CSF of MS patients (94% v. 0) is an unequivocal basis for the use of tests for the presence this protein in MS diagnostics.

The presence of the Nogo-A fragment in the CSF may also explain the inability of axons to regenerate in MS disease centers in the brain. Thus, it is also possible to use this Nogo-A protein fragment to produce blockers of free Nogo-A which should lead to decreased axonal injury and increased regeneration of changes due to MS. This activity of Nogo-A blockers would facilitate new methods of MS treatment and thus a limitation of disability resultant from MS.

To summarise, it should be stated that the specific localisation of the soluble Nogo-A fragment in the CSF of MS patients facilitates the use of the detection of said Nogo-A fragment as a diagnostic test for MS and the use of Nogo-A blockers as a method of treating MS.

### Examples

The research made use of the following antibodies: goat anti-Nogo (sc-11033), anti-Nogo-A (sc-11032), anti-Nogo-66 receptor (Nogo-R), anti-MAG and anti-MOG; anti-goat conjugated with HRP, anti-rabbit conjugated with HRP; blocking peptides as well as the anti-Notch-1, anti-Notch-3 and anti-Jagged from Santa Cruz (Santa Cruz, CA); antibodies recognising the myelin basic protein - MBP were purchased from Sigma (Sigma, Poznan, Polska). Chemicon (Chemicon, CA, USA) supplied anothe rabbit antibody against Nogo-A (AB5888). Peptides PLP139-151 and 178-191, peptide MBP 87-99, and peptide MOG 35-55 were synthesized at the Albert Einstein College of Medicine, New York, and the MBP protein was purchased from Sigma. All of said reagents were used to block the anti-Nogo-A antibody. The anti-Nogo globulin recognizes the C-terminal peptide, common to all Nogo isoforms: A, B and C. Anti-Nogo-A recognizes the N-terminal region of Nogo-A, characteristic for only that isoform.

CSF was collected and tested from 114 MS patients, 115 other neural disorder (OND) patients, 18 meningitis patients and 11 patients with clinically isolated syndrome (CIS). CSF samples were initially centrifuged (6000 rpm) and then frozen at - 80°C until needed. The protein content of each sample was assessed using a GeneQuant system (Pharmacia), and the same amount of protein (3µg) was placed in each lane and electrophoresed.

All patients afflicted with recognized MS fulfilled McDonald criteria for clinical MS. The patients were divided into 2 groups according to disease progression: 1. remitting relapsing MS (RR-MS) (n=103) and 2. secondary progressive (SP-MS) (n=11). Among RR-MS patients, 33 were tested during remission relapse. 31 patients had visible changes in MRI images shortly prior to CSF sampling, and 13 had enhanced changes following contrasting. The mean age of RR-MS patients was 35·4±9·2 years, and the average disease duration was 5·2±3·9 years. The average degree of disability was 3·5±2·5 on the EDDS scale. The average age of SP-MS patients was 44·2±5·9 years and average disease duration was 7·8±4·5 years. The mean degree of disability was 3·75±1·0 on the EDDS scale. The CIS group contained 11 patients, with an average age of 25±2,7 years. Clinical signs of in this group included extraocular inflammation of the optic nerve, spinal injury and other isolated neurological symptoms. The mean age of meningitis patients was 41±13 years. One of the patients was diagnosed with a tuberculous meningitis, 4 patients with bacterial meningitis, arboviral meningitis in 3 patients, and meningitis due to other viruses in 10 patients. The mean age of OND patients was 47±21 years, and the following were recognized intensive headaches exclusive of subarachnoid haematoma (49), ischaemia (35), spinal pain syndromes (17), Creuzfeldt-Jakob disease (3), epilepsy (3), cranial nerve trauma (8) chronic polyneuropathy (7) and Device's syndrome (3). Among the group of patients with other autoimmunological diseases of the CNS were 2 patients with Behcet's disease, 4 patients with SLE, 3 patients with cerebral vascularitis 1 and one patient with a neurosarcoid.

Brain tissues were obtained from the autopsies of 3 MS patients (2 women and 1 man) and 2 patients with brain ischaemia (1 woman and 1 man).

Western blot analysis was performed using fragments from demyelinating disease samples confirmed using microscopy and haematoxilin/eosin staining. For the Western blots, 10 g of brain tissue were homogenized in 40 ml of hypotonic buffer (30mM NaHCO₃ and 0,5 mM PMSF, pH 7,1), and centrifuget at 100,000 x g. Protein content was evaluated using a GeneQuant system (Pharmacia), and identical amounts of protein were applied in each electrophoresis lane and then used in Western blotting.

Tissue lysate and CSF proteins were electrophoresed in SDS-PAGE gels and transferred onto PVDF membranes, blocked in 5% milk in PBS-Tween 20 overnight at 4°C. The membrane was incubated with primary antibodies against Nogo, Nogo-A, Nogo-R, MAG, MOG and MBP (1µg/ml in 5% NFDM in PBS-Tween) for 1 h. In blocking experiments, the anti-Nogo-A antibody was incubated with a blocking peptide for 2h at room temperature and subsequently used in detection on membranes. After multiple washes the membrane was incubated for 1h with a secondary antibody. After multiple washes the membrane was incubated with ECL Plus and the chemiluminescence wwas evaluated using a camera.

Statistical significance was evaluated using Fisher's test. The results of the above described experiments illustrating the embodiment of claimed aspects are presented in the table below, as well as in Figures 1-3.

**Table 1. The presence of the Nogo-A protein fragment according to the present invention in the CSF of various patient groups using Western blotting**

| Patients | Nogo-A | |
|---|---|---|
| | positive result | negative result |
| MS | 110 | 4 |
| CIS (Clinically isolated syndrome) | 11 | 0 |
| Meningitis | 0 | 18 |
| intensive headaches not related to subarachnoid haematoma | 0 | 49 |
| Creuzfeldt-Jakob disease | 0 | 3 |
| epilepsy | 0 | 3 |
| back pain syndromes | 0 | 17 |
| ischaemia | 0 | 35 |
| cranial nerve trauma | 0 | 8 |
| Behcet's disease | 0 | 2 |
| SLE | 0 | 4 |
| cerebral vascularitis | 0 | 3 |
| neurosarkoid | 0 | 1 |
| CIDP | 0 | 7 |
| Devic's syndrome | 0 | 3 |

## Claims

1. An isolated Nogo-A protein fragment with a molecular mass of 20 kDa and possessing an amino-acid sequence containing the peptide sequence SEQ ID No.1.

2. An isolated polynucleotide molecule encoding the Nogo-A protein fragment with a molecular mass of 20 kDa and possessing an amino-acid sequence containing the peptide sequence SEQ ID No.1.

3. A polynucleotide molecule according to claim 2 encoding the peptide consisting of SEQ ID No.1.

4. A method of diagnosing multiple sclerosis or immune demyelinating polyneuropathy, **characterized in that** a CSF sample from a patient is examined for the presence of the Nogo-A protein fragment with a molecular mass of 20 kDa and possessing an amino-acid sequence containing the peptide sequence SEQ ID No.1, wherein its presence is indicative of multiple sclerosis or immune demyelinating polyneuropathy.

5. A method according to Claim 4, **characterized in that** the presence of the Nogo-A protein fragment is determined using an antibody specific against this fragment.

6. A method of obtaining an antibody for the diagnosis or treatment of multiple sclerosis or immune demyelinating polyneuropathy encompassing the immunization of a mammal with a specific antigen as well as the isolation of specific antibodies against the antigen, **characterized in that** the immunization is performed using the Nogo-A protein fragment with a molecular mass of 20 kDa and possessing an amino-acid sequence containing the peptide sequence SEQ ID No.1 or a fragment of said protein containing SEQ ID No.1, preferentially a peptide consisting of SEQ ID No.1.

7. An antibody manufactured using the method according to Claim 6 for use in the diagnostics or therapy of multiple sclerosis or immune demyelinating polyneuropathy.

8. Use of a specific antibody against the Nogo-A protein fragment with a molecular mass of 20 kDa and possessing an amino-acid sequence containing the peptide sequence SEQ ID No.1 or an antibody specific against a fragment of this protein containing SEQ ID No.1 in the manufacture of a preparation for the treatment or diagnosis of multiple sclerosis or immune demyelinating polyneuropathy.

9. Use according to Claim 8, **characterized in that** the antibody used is specific against a peptide consisting of SEQ ID No.1.

10. Use of the Nogo-A protein fragment with a molecular mass of 20 kDa and possessing an amino-acid sequence containing the peptide sequence SEQ ID No.1 or a fragment of this protein containing SEQ ID No.1 in the manufacture of a preparation for the treatment or diagnosis of multiple sclerosis or immune demyelinating polyneuropathy.

11. Use according to Claim 10, **characterized in that** the protein fragment used is a peptide consisting of SEQ ID No.1

## Patentansprüche

1. Ein isoliertes Nogo-A-Proteinfragment mit einem Molekulargewicht von 20 kDa, das eine Aminosäuresequenz besitzt, die die Peptidsequenz SEQ ID NO:1 enthält.

2. Ein isoliertes Polynukleotidmolekül, das für das Nogo-A-Proteinfragment mit einem Molekulargewicht von 20 KDa kodiert, das eine Aminosäuresequenz besitzt, die die Peptidsequenz SEQ ID NO:1 enthält.

3. Ein Polynukleotldmolekül nach Anspruch 2, das für das Peptid kodiert, das aus SEQ ID NO:1 besteht.

4. Ein Verfahren zur Diagnose von multipler Sklerose oder demyelinisierender immunpolyneuropathie, dadurch charakterisiert, dass eine CSF-Probe eines Patienten auf das Vorhandensein des Nogo-A-Proteinfragments mit einem Molekulargewicht von 20 kDa untersucht wird, das eine Aminosäuresequenz besitzt, die die Peptidsequenz SEQ ID NO:1 enthält, wobei dessen Vorhandensein auf multiple Sklerose oder demyelinisierende Immunpolyneuropathie hinweist,

5. Ein Verfahren nach Anspruch 4, dadurch charakterisiert, dass das Vorhandensein das Nogo-A-Proteinfragments mithilfe eines für dieses Fragment spezifischen Antikörpers bestimmt wird.

6. Ein Verfahren zu m Herstellen eines Antikörpers für die Diagnose oder Behandlung von multipler Sklerose oder demyelinislerender Immunpolyneuropathie, umfassend die Immunisierung eines Säugetiers mit einem spezifischen Antigen sowie die Isolierung von spezifischen Antikörpern gegen das Antigen, dadurch charakterisiert, dass die Immunisierung mithilfe das Nogo-A-Proteinfragments mit einem Molekulargewicht von 20 kDa, das eine Aminosäuresequenz besitzt die die Peptidsequenz SEQ ID NO:1 enthält, oder eines Fragments dieses Proteins, das SEQ ID NO:1 enthält, vorzugsweise eines Peptids, das aus SEQ ID NO: 1 besteht, durchgeführt wird.

7. Ein Antikörper, der mithilfe des Verfahrens nach Anspruch 6 hergestellt wurde, zur Verwendung in der Diagnostik oder Therapie von multipler Sklerose oder demyelinisierender Immunpolyneuropathie.

8. Verwendung eines spezifischen Antikörpers gegen das Nogo-A-Proteinfragment mit einem Molekulargewicht von 20 kDa. das eine Aminosäuresequenz besitzt, die die Peptidsequenz SEQ ID NO:1 enthält, oder eines Antikörpers, der spezifisch für ein Fragment dieses Proteins, das SEQ ID NO:1 enthält, ist, zur Herstellung einer Zubereitung für die Behandlung oder Diagnose von multipler Sklerose oder demyelinisierender immunpolyneuropathie.

9. Verwendung nach Anspruch 8, dadurch charakterisiert, dass der verwendete Antikörper spezifisch für ein Peptid ist, das aus SEQ ID NO:1 besteht.

10. Verwendung des Nogo-A-Proteinfragments mit einem Molekulargewicht von 20 kDa, das eine Aminosäuresequenz besitzt, die die Peptidsequenz SEQ ID NO:1 enthält, oder eines Fragments dieses Proteins, das SEQ ID NO.1 enthält, zur Herstellung einer Zubereitung für die Behandlung oder Diagnose von multipler Sklerose oder demyelinisierender Immunpolyneuropathie.

11. Verwendung nach Anspruch 10, dadurch charakterisiert, dass das verwendete Proteinfragment ein Peptid ist, das aus SEQ ID ND:1 besteht.

## Revendications

1. Fragment de protéine Nogo-A isolé avec une masse moléculaire de 20 kDa et possédant une séquence d'acides aminés contenant la séquence peptidique SEQ ID N° 1.

2. Molécule polynucléotidique isolée codant pour le fragment de protéine Nogo-A avec une masse moléculaire de 20 kDa et possédant une séquence d'acides aminés contenant la séquence peptidique SEQ ID N° 1.

3. Molécule polynucléotidique selon la revendication 2 codant pour le peptide constitué par SEQ ID N° 1.

4. Procédé de diagnostic de la sclérose en plaques ou de la polyneuropathie démyélinisante immune, **caractérisé en ce qu'**un échantillon de liquide céphalorachidien provenant d'un patient est examiné pour détecter la présence du fragment de protéine Nogo-A avec une masse moléculaire de 20 kDa et possédant une séquence d'acides aminés contenant la séquence peptidique SEQ ID N° 1, dans lequel sa présence indique une sclérose en plaques ou une polyneuropathie démyélinisante immune.

5. Procédé selon la revendication 4, **caractérisé en ce que** la présence du fragment de protéine Nogo-A est déterminée à l'aide d'un anticorps spécifique contre ce fragment.

6. Procédé d'obtention d'un anticorps pour le diagnostic ou le traitement de la sclérose en plaques ou de la polyneuropathie démyélinisante immune comprenant l'immunisation d'un mammifère avec un antigène spécifique ainsi que l'Isolement d'anticorps spécifiques contre l'antigène, **caractérisé en ce que** l'immunisation est réalisée à l'aide du fragment de protéine Nogo-A avec une masse moléculaire de kDa et possédant une séquence d'acides aminés contenant la séquence peptidique SEQ ID N° 1 ou un fragment de ladite protéine contenant SEQ ID N° 1, de préférence un peptide constitué par SEQ ID 1.

7. Anticorps fabriqué à l'aide du procédé selon la revendication 6 destiné à être utilisé dans le diagnostic ou la thérapie de la sclérose en plaques ou de la polyneuropathie démyélinisante immune.

8. Utilisation d'un anticorps spécifique contre le fragment de protéine Nogo-A avec une masse moléculaire de 20 kDa et possédant une séquence d'acides aminés contenant la séquence peptidique SEQ ID N° 1 ou un anticorps spécifique contre un fragment de cette protéine contenant SEQ ID N° 1 dans la fabrication d'une préparation destinée au traitement au au diagnostic de la sclérose en plaques ou de la polyneuropathie démyélinisante immune.

9. Utilisation selon la revendication 8, **caractérisée en ce que** l'anticorps utilisé est spécifique contre un peptide constitué par SEQ ID N° 1.

10. Utilisation du fragment de protéine Nogo-A avec une masse moléculaire de 20 kDa et possédant une séquence d'acides aminés contenant la séquence peptidique SEQ ID N° 1 ou un fragment de cette protéine contenant SEQ ID N° 1 dans la fabrication d'une préparation destinée au traitement ou au diagnostic de la sidérose en plaques ou de la polyneuropathie démyélinisante immune.

11. Utilisation selon la revendication 10, **caractérisée en ce que** la fragment de protéine utilisé est un peptide constitué par SEQ ID N° 1.
